# FASCICULE DE BREVET EUROPEEN

(11) **EP 1 066 049 B1**
(45) Date de publication et mention de la délivrance du brevet: **28.05.2003**
(21) Numéro de dépôt: 99910418.5
(22) Date de dépôt: 22.03.1999
(51) Int. Cl.: A61K 38/09, A61K 38/31, A61K 9/14, A61K 47/26

(54) **COMPOSITIONS PHARMACEUTIQUES DESTINEES A LA LIBERATION PROLONGEE DE PEPTIDES ET LEUR PROCEDE DE PREPARATION**
PHARMAZEUTISCHE ZUSAMMENSETZUNGEN FÜR DIE VERZÖGERTE FREISETZUNG VON PEPTIDEN UND VERFAHREN ZU DEREN HERSTELLUNG
PHARMACEUTICAL COMPOSITIONS FOR PROLONGED PEPTIDE RELEASE AND PREPARATION METHOD

(30) Priorité: 25.03.1998 FR 9803667
(43) Date de publication de la demande: 10.01.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: PELLET, Marc, F-27160 Condé sur Iton (FR); BISMUTH, Frédéric, F-28100 Dreux (FR)
(74) Mandataire: Bourgouin, André
(86) Numéro de dépôt international: FR9900667
(87) Numéro de publication internationale: WO99048517

(56) Documents cités:
- WO-A-90/13285
- US-A- 5 595 760

## Description

L'invention concerne de nouvelles compositions pharmaceutiques destinées à la libération prolongée de peptides et leur procédé de préparation.

Il avait déjà été décrit dans le brevet américain 5,595,760 des compositions pharmaceutiques solides et servi-solides destinées à la libération prolongée de peptides composées d'un sel hydrosoluble et gélifiable de peptide associé éventuellement à un excipient monomère adapté. Après administration à un patient, ces compositions gélifient et permettent une libération prolongée sur une période d'au moins trois jours.

Ces compositions apportaient un avantage considérable par rapport à l'art antérieur au niveau de leur simplicité de fabrication et d'utilisation.

La demanderesse vient de découvrir de façon inattendue qu'on pouvait obtenir des compositions perfectionnées qui, tout en utilisant le même principe, permettent d'obtenir une libération plus lente que les compositions classiques, et pouvant dans certains cas atteindre un, deux, trois mois ou plus. En particulier, le pic initial (ou "burst" en anglais) est réduit.

De plus, les compositions de l'invention sont plus faciles à préparer. Notamment, le temps de broyage du peptide et la force nécessaire pour le malaxage peuvent être largement réduits. Les compositions de l'invention présentent également des caractéristiques plus homogènes.

Outre les avantages précédemment mentionnés, certaines de ces compositions présentent l'avantage, à quantité de peptide égale, de nécessiter une force d'injection moindre et offrent donc un meilleur confort d'utilisation. Ceci permet donc d'utiliser des seringues ayant des aiguilles de diamètre inférieur à celui qui serait nécessaire pour les compositions équivalentes de l'art antérieur.

On constate par ailleurs que ces compositions donnent de très bons résultats lors des tests *in vivo* et que les écarts expérimentaux individuels sont réduits, ce qui permettra de traiter efficacement une plus grande proportion de patients.

Tous ces avantages sont obtenus en donnant au peptide une surface spécifique plus élevée que dans les compositions non matricielles (gélifiables) connues de l'homme du métier et décrites dans le brevet américain 5,595,760. Les compositions gélifiables selon l'invention utilisent de préférence des peptides dont la surface spécifique a été portée à au moins 4 m²/g, et plus préférentiellement à 8 m²/g ou plus, cette caractéristique leur conférant un profil de libération plus lent et régulier. Les compositions selon l'invention sont obtenues en utilisant un procédé de lyophilisation particulier comprenant une phase de congélation-éclair d'une solution du peptide, procédé qui sera décrit plus loin.

L'invention concerne donc tout d'abord une composition pharmaceutique solide ou semi-solide comprenant un sel soluble et gélifiable de peptide associé éventuellement à un excipient adapté, ladite composition pharmaceutique étant caractérisée en ce que le sel de peptide possède une surface spécifique élevée et en ce qu'une fois injectée à un patient elle forme au contact des matières corporelles un gel chez ce patient, ledit gel étant capable de relarguer le peptide sur une durée prolongée et au moins égale à 15 jours.

Par surface spécifique élevée, on entend une surface spécifique supérieure à celle qui serait obtenue par une lyophilisation mettant en oeuvre une congélation lente d'une solution d'un sel de peptide. Par congélation lente, on entend une congélation n'étant pas une congélation éclair telle que décrite plus loin ou dans la demande de brevet PCT WO 98/47489.

De préférence, le sel de peptide possède une surface spécifique au moins égale à 8 m²/g. et en ce qu'une fois injectée à un patient elle forme au contact des matières corporelles un gel chez ce patient, ledit gel étant capable de relarguer le peptide sur une durée prolongée et au moins égale à 15 jours.

L'invention concerne donc de préférence une composition pharmaceutique solide ou semi-solide comprenant un sel soluble et gélifiable de peptide associé éventuellement à un excipient adapté, ladite composition pharmaceutique étant caractérisée en ce que le sel de peptide possède une surface spécifique au moins égale à 4 ou 5 m²/g, et plus préférentiellement à 8 m²/g et en ce qu'une fois injectée à un patient elle forme au contact des matières corporelles un gel chez ce patient, ledit gel étant capable de relarguer le peptide sur une durée prolongée et au moins égale à 15 jours.

Par peptide, on entend aussi bien peptide que protéine. On pourra notamment choisir les sels de peptides utilisables pour l'invention dans un groupe composé des sels des substances suivantes : la triptoréline, le lanréotide, l'octréotide (tel que décrit par exemple dans le brevet européen EP 29 579), un composé ayant une activité LH-RH tel que la triptoréline, la goséréline, la leuproréline, la buséréline, un antagoniste de LH-RH, un antagoniste de GPIIb/IIIa, un composé ayant une activité similaire à un antagoniste de GPIIb/IIIa, l'érythropoiétine (EPO) ou un de ses analogues, les différents interférons α, l'interféron β ou γ, la somatostatine, un dérivé de la somatostatine tel que décrit dans le brevet européen EP 215 171, un analogue de la somatostatine tel que décrit dans le brevet américain US 5,552,520 (ce brevet comporte lui-même une liste d'autres brevets décrivant des analogues de la somatostatine qui sont incorporés par référence à la présente demande), l'insuline, une hormone de croissance (GH), un facteur libérateur d'hormone de croissance (GRF), un peptide libérateur d'hormone de croissance (GHRP), un facteur de croissance épidermique (EGF), une hormone mélanocyte-stimulante (MSH), une hormone libératrice de thyrotropine (TRH) ou un de ses dérivés, une hormone stimulant la thyroïde (TSH), une hormone lutéinisante (LH), une hormone stimulant les follicules (FSH), une hormone parathyroïdienne (PTH) ou un de ses dérivés, un hydrochlorure de lysozyme, un peptide relié à l'hormone parathyroïdienne (PTHrp), un fragment de peptide à N terminal (position 1―>34) de l'hormone PTH humaine, la vasopressine ou un de ses dérivés, l'oxytocine, la calcitonine, un dérivé de la calcitonine ayant une activité similaire à celle de la calcitonine, un peptide relié au gène de la calcitonine (CGRP), le glucagon, un peptide similaire au glucagon (GLP), la gastrine, un peptide libérateur de gastrine (GRP), la sécrétine, la pancréozymine, la cholécystokinine, l'angiotensine, le lactogène du placenta humain, la gonadotropine chorionique humaine (HCG), l'enképhaline, un dérivé de l'enképhaline, le facteur stimulateur de colonies (CSF), l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombésine ou un de ses dérivés tels que décrits dans le brevet américain US 5,552,520 (ce brevet comporte lui-même une liste d'autres brevets décrivant des dérivés de la bombésine qui sont incorporés par référence à la présente demande), la prolactine, la neurotensine, la dynorphine, la caeruléine, la substance P, l'urokinase, l'asparaginase, la bradykinine, la kallikréine, le facteur de croissance nerveuse, un facteur de coagulation sanguine, la polymixine B, la colistine, la gramicidine, la bacitracine, un peptide stimulant la synthèse protéique, un antagoniste de l'endothéline ou un de ses dérivés, un polypeptide intestinal vasoactif (VIP), l'hormone adrénocorticotropique (ACTH) ou un de ses fragments, un facteur de croissance dérivé des plaquettes (PDGP), une protéine morphogénétique osseuse (BMP), un polypeptide activant l'adénylatecyclase pituitaire (PACAP), le neuropeptide Y (NPY), le peptide YY (PYY), un polypeptide inhibiteur gastrique (GIP). Tout sel hydrosoluble de peptide ou de protéine pourra également être utilisé par l'homme du métier s'il le juge utile.

De préférence, le sel de peptide utilisé pour l'invention sera choisi dans un groupe comprenant des sels de la somatostatine ou de ses analogues, en particulier l'acétate de lanréotide ou l'acétate d'octréotide, des sels de la triptoréline, en particulier l'acétate de triptoréline, des sels de la calcitonine ou de ses analogues, des sels d'analogues de l'hormone LH-RH, des sels des hormones GH, GRF, PTH ou du peptide PTHrp, et des analogues de ces derniers.

Les sels du peptide utilisables pour l'invention sont de préférence des sels pharmaceutiquement acceptables d'acides organiques, tels que ceux des acides acétique, lactique, malique, ascorbique, succinique, benzoïque, méthanesulfonique ou toluènesulfonique, ou encore des sels pharmaceutiquement acceptables d'acides inorganiques, tels que ceux des acides chlorhydrique, bromhydrique, iodhydrique, sulfurique ou phosphorique. Ils pourront en particulier être des acétates dudit peptide. La solubilité du sel du peptide doit toutefois être assez importante pour permettre la congélation du sel de peptide avec une faible quantité de solvant.

De préférence, la surface spécifique du sel de peptide sera au moins égale à 4 ou 5 m²/g. Plus préférentiellement, le sel de peptide aura une surface spécifique au moins égale à 10 ou 15 m²/g. Tout préférentiellement, le sel de peptide aura une surface spécifique au moins égale à 20 m²/g, voire 30 m²/g. Ces surfaces spécifiques peuvent être obtenues en utilisant les procédés décrits plus loin ou dans la demande PCT WO 98/47489.

Ladite composition solide ou semi-solide pourra comprendre de 0 à 30 % d'un excipient. Des excipients utilisables pour l'invention sont des excipients pharmaceutiquement acceptables facilitant la préparation des compositions de l'invention et/ou leur administration. Les excipients choisis devront être hydrosolubles et biodégradables au contact des matières corporelles. Il pourra notamment s'agir de polyalcools tel que le mannitol et le sorbitol, de sucres tels que le glucose et le lactose, de surfactants, de solvants organiques ou de polysaccharides. Toutefois, ces excipients ne seront pas des polymères matriciels, comme par exemple des polymères de type PLGA.

Pour préparer les compositions pharmaceutiques de l'invention, on utilisera un procédé caractérisé en ce qu'il comprend une étape de lyophilisation comprenant une trempe rapide d'une solution diluée du sel de peptide dans un milieu de température inférieure à -50 °C.

Par trempe rapide, il faut entendre une mise en contact avec un milieu à basse température provoquant une congélation instantanée de la solution de substance hydrosoluble.

Par solution diluée du sel de peptide, on entend une solution ayant une concentration dudit sel de peptide inférieure à la moitié de la concentration de saturation, et de préférence inférieure à un quart de ladite concentration de saturation lorsque celle-ci est au moins égale à 200 g/l. Ce procédé permet d'obtenir un sel de peptide présentant une surface spécifique élevée.

Pour la lyophilisation, on pourra par exemple congeler la solution dans un plateau baignant dans un bac d'azote liquide, avant de procéder à la lyophilisation proprement dite.

De préférence, la trempe rapide sera réalisée en versant une solution diluée du sel de peptide sur une plaque métallique à très basse température. La température de la plaque sera de préférence inférieure à -70 °C, et plus préférentiellement inférieure à -80 °C, voire -120 °C. Cette trempe permet d'obtenir un sel de peptide possédant une surface spécifique très élevée telle que décrite précédemment.

Plus préférentiellement, afin d'obtenir une surface spécifique maximale, la trempe rapide de la solution sera précédée d'une micronisation de la solution de substance active.

Si une surface spécifique supérieure à 10 m²/g est nécessaire, il sera préférable de recourir au procédé incluant une étape de micronisation. De préférence, la surface spécifique obtenue pour la substance active après lyophilisation sera supérieure à 15 m²/g. Encore plus préférentiellement, cette surface spécifique sera supérieure à 20 m²/g, voire 30 m²/g. Les surfaces spécifiques élevées seront particulièrement utiles car la force nécessaire à l'injection sera plus faible et le diamètre de l'aiguille utilisée pour l'injection pourra être moins important.

Par exemple, pour obtenir une surface spécifique très élevée, on pourra choisir d'atomiser la solution en la pulvérisant à travers un atomiseur sur une plaque à très basse température. La température de la plaque sera inférieure à -50 °C, de préférence inférieure à -70 °C, et plus préférentiellement encore inférieure à -80 °C, voire -120 °C. Cette température pourra être atteinte par exemple en faisant tremper la plaque métallique dans un milieu à très basse température comme par exemple de l'azote liquide. Selon une variante préférée de l'invention, la plaque métallique est creuse et la solution est pulvérisée à l'aide d'un atomiseur à l'intérieur de ladite plaque.

D'autres techniques de congélation sont envisageables pour obtenir une surface spécifique très importante, par exemple l'atomisation de la solution de substance active dans un bain de non-solvant du sel de peptide préalablement réfrigéré. Comme non-solvant, on préférera un gaz liquéfié comme par exemple l'azote liquide.

Une autre possibilité est de congeler la solution de sel de peptide sur un plateau tournant réfrigéré ("drum-freezing"). Comme indiqué précédemment, cette congélation sera de préférence précédée d'une micronisation de la solution de sel de peptide.

La surface spécifique de la substance active est un facteur favorable pour obtenir une libération sur une période prolongée. En effet, des particules d'un sel de peptide de même taille mais de surfaces spécifiques différentes donneront des résultats tout à fait différents.

Pour faire varier les surfaces spécifiques obtenues, on pourra faire varier les conditions de congélation de la solution de substance active en jouant sur différents paramètres tels que par exemple la vitesse de congélation ou la concentration de la solution.

La lyophilisation se fera dans des conditions classiques et connues de l'homme du métier. A l'issue de la lyophilisation, le sel de peptide est incorporé, éventuellement avec un excipient, dans une composition pharmaceutique solide ou serai-solide telle que décrite précédemment. Cette composition solide ou semi-solide peut être mélangée avec de l'eau comme décrit dans le brevet américain 5,595,760, en tenant notamment compte du fait que l'eau peut être présente en une quantité inférieure à 50 % de la quantité nécessaire pour dissoudre entièrement le sel de peptide, ladite quantité devant en outre être adaptée pour donner une consistance semi-solide à ladite composition.

De préférence, lorsque c'est possible, la quantité d'eau ajoutée sera inférieure à 30 %, et plus préférentiellement inférieure à 10 % de la quantité nécessaire pour dissoudre entièrement le sel de peptide.

La proportion de peptide dans les compositions selon l'invention sera déterminée par la durée de libération que l'on souhaite obtenir. Mais elle ne saura excéder une valeur maximale qui correspond à la concentration limite pour pouvoir injecter la composition solide ou semi-solide avec une seringue disposant d'une aiguille d'un diamètre usuel. On pourra faire varier la surface spécifique du peptide afin d'élever ladite concentration limite si besoin est ; plus la surface spécifique du peptide sera élevée, moins la force d'injection sera importante, ce qui permettra de réduire le diamètre de l'aiguille nécessaire pour l'injection.

Par exemple, pour de l'acétate de lanréotide d'une surface spécifique élevée (par exemple au moins égale à 4 m²/g) obtenue par un procédé de lyophilisation comprenant une étape de congélation éclair, on pourra utiliser des compositions semi-solides possédant des concentrations comportant 25 ou 30 % en poids d'acétate de lanréotide dans l'eau (soit 20,5 ou 24,6 % en poids de lanréotide pur). De telles compositions pourront aisément être injectées avec des aiguilles d'un diamètre interne de l'ordre de 1 mm et d'une longueur de l'ordre de 32 mm.

De préférence, les compositions de l'invention à base d'acétate de lanréotide comprendront de 20 à 35 %, et plus préférentiellement de 25 à 30 % en poids d'acétate de lanréotide.

Le malaxage de la composition solide et de l'eau pour donner des compositions senti-solides est de préférence effectué dans un dispositif constitué de deux seringues reliées entre elles. Par exemple, le sel de peptide est introduit dans l'une des seringues et conditionné sous vide, l'eau est introduite dans l'autre seringue, et le mélange est homogénéisé par va-et-vient des deux pistons. A cet effet, l'homme du métier pourra aussi utilement consulter la demande de brevet PCT WO 97/46202.

Comme indiqué ci-dessus, les compositions semi-solides selon l'invention trouvent leur usage de préférence dans le domaine pharmaceutique. Les compositions selon l'invention pourront être injectées à un patient, par exemple en utilisant les dispositifs décrits dans le brevet américain 5,595,760.

Une fois injectées à un patient, les compositions semi-solides selon l'invention forment au contact des matières corporelles un gel chez ce patient, ledit gel étant capable de relarguer le peptide sur une durée prolongée et au moins égale à 15 jours. De préférence, la durée du relargage sera au moins égale à 1 mois, et plus préférentiellement à 2, voire 3 mois.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

### EXEMPLES :

### Méthodes :

### Mesure de la surface spécifique

Pour tous les exemples qui suivent, la surface spécifique du sel de peptide a été déterminée par la méthode dite méthode B.E.T. (absorption d'une monocouche d'azote sur la substance active), méthode bien connue de l'homme du métier.

### Malaxage du peptide et de l'eau

Pour tous les exemples qui suivent, le sel de peptide et l'eau sont malaxés dans un dispositif constitué de deux seringues de 50 ml reliées entre elles. Le sel de peptide est introduit dans l'une des seringues et conditionné sous vide, l'eau est introduite dans l'autre seringue, et le mélange est homogénéisé par va-et-vient des deux pistons.

### Exemple 1 :

De l'acétate de lanréotide de surface spécifique 0,61 m²/g est mis en solution dans de l'eau à une concentration de 30 g/l et est congelé en versant la solution aqueuse obtenue dans un plateau métallique creux refroidi à l'extérieur par de l'azote liquide. Le sel de peptide est ainsi congelé. On procède ensuite à la lyophilisation et récupère de l'acétate de lanréotide de surface spécifique 5,41 m²/g.
3 g d'acétate de lanréotide ainsi obtenu sont mélangés avec 6,927 ml d'eau pour donner une pâte semi-solide. Le mélange est ensuite malaxé comme indiqué plus haut pour donner 10,927 g d'une composition semi-solide homogène et compacte. Cette composition est directement utilisable pour une injection au sujet à traiter.

### Exemple 2 :

9,0 g d'acétate de lanréotide de surface spécifique 1,73 m²/g sont dissous dans 300 ml d'eau. Cette solution est ensuite pulvérisée à l'aide d'un atomiseur dans un plateau métallique creux dont le fond trempe dans de l'azote liquide. Le sel de peptide est ainsi congelé. On procède ensuite à la lyophilisation et récupère 8,7 g d'acétate de lanréotide de surface spécifique 28,2 m²/g.
3 g d'acétate de lanréotide ainsi obtenu sont mélangés avec 7,183 ml d'eau pour donner une pâte semi-solide. Le mélange est ensuite malaxé comme indiqué plus haut pour donner 10,183 g d'une composition semi-solide homogène et compacte. Cette composition est directement utilisable pour une injection au sujet à traiter.

### Exemples 3 et 4 :

Le même protocole est utilisé pour ces deux exemples :

5 g d'acétate de lanréotide sont dissous dans de l'eau stérile pour donner la concentration choisie à la solution. Cette solution est atomisée à l'aide d'un pulvérisateur de 500 ml, dont le jet est réglé de façon à obtenir les goutelettes les plus fines possibles. Les gouttelettes obtenues sont projetées dans un plateau dont le fond trempe dans l'azote liquide. Deux sondes de température sont préalablement introduites dans le plateau afin de suivre l'évolution de la température du produit.
Une fois le produit congelé, le plateau est introduit dans le lyophilisateur dont la plaque est à environ -54 °C.
On laisse s'équilibrer la température des produits et de la plaque pendant 1 heure. Puis on passe à la phase de sublimation (la température de la plaque est alors fixée à 20 °C et la pression dans la cuve à 100 µbar). Cette phase dure environ 30 heures. La température finale moyenne du produit est environ 13 °C. La dessiccation secondaire qui suit (pression dans la cuve portée à 50 µbar) dure environ 24 heures. La température finale moyenne du produit est de 20 °C.

Les caractéristiques des réactifs engagés et des produits obtenus sont résumés dans le tableau ci-après :

| Caractéristiques | Exemple 3 | Exemple 4 |
|---|---|---|
| Masse d'acétate de lanréotide engagée (g) | 5,00 | 5,00 |
| Concentration de la solution (g/l) | 30 | 10 |
| Masse d'acétate de lanréotide récupérée | 4,54 | 4,10 |
| Surface spécifique obtenue (m²/g) | 36 | 43 |

Comme l'acétate de lanréotide des exemples 1 et 2, l'acétate de lanréotide des exemples 3 et 4 peut être incorporé à des compositions pharmaceutiques semi-solides par simple mélange avec une quantité d'eau adaptée.

### Etude des propriétés de compositions selon l'invention

Trois tests ont été réalisés. Le premier porte sur la force nécessaire à l'injection d'une dose de composition obtenue selon l'exemple 2, le second sur le profil de libération in vitro de la même composition et le troisième sur le profil de libération chez le chien des compositions des exemples 1 et 2 par rapport à celui obtenu pour une composition analogue mais avec un peptide de faible surface spécifique.

### Référence

Afin de servir de référence pour la mesure de la force de libération et le test in vitro, une composition réalisée selon le protocole suivant a été choisie :
De l'acétate de lanréotide est mis en solution dans de l'eau pour donner une solution de concentration 30 g/l, laquelle est versée dans un plateau creux préalablement plongé dans de l'azote liquide. L'acétate de lanréotide ainsi congelé est ensuite lyophilisé et incorporé dans une composition solide comme décrit dans l'exemple 2 ci-dessus, 6,817 ml d'eau étant ajoutés à 3 g d'acétate de lanréotide pour donner 9,817 g de composition semi-solide.

### Mesure de la force nécessaire à l'injection

On mesure à l'aide d'un dynamomètre la force à appliquer sur le piston de la seringue pour le faire progresser par rapport au déplacement du piston (quantité de composition servi-solide injectée : environ 280 mg ; tout comme l'exemple 2, la référence contient environ 0,25 mg d'acétate de lanréotide par mg de composition semi-solide). En exprimant le déplacement du piston en mm en fonction de la force appliquée en N, on obtient un profil triphasique, duquel sont tirées 8 valeurs remarquables servant à calculer une valeur moyenne pour la force nécessaire à l'injection. La valeur retenue pour chaque test est la moyenne de 5 mesures effectuées sur la même composition.

### Profil de libération in vitro

Afin d'obtenir des résultats significatifs, chaque composition testée est répartie en 6 échantillons, et la valeur retenue est la moyenne obtenue sur les 6 essais.
Dans chaque cas, la composition semi-solide à tester est placée dans un tube de dialyse cylindrique équipé d'une membrane synthétique semi-perméable. Les deux extrémités du tube sont fermées. Ce tube est placé dans 20 ml de solution acqueuse de NaCl à 0,9 %, la température étant fixée à 37 °C. Le milieu est agité (agitateur magnétique).

Une demi-heure, 1 h, 2 h, 3 h, 4 h, 24 h, 48 h et 72 h après le début du test, des prélèvements de la solution de NaCl sont effectués et la teneur en lanréotide est déterminée par dosage UV (longueur d'onde : 280 nm).
A la fin du test (après 96 h pour la référence), la partie rémanente du peptide contenue dans le tube de dialyse est dosée afin d'exprimer les résultats en proportion de peptide libérée par rapport à la quantité totale initiale.

### Résultats

Les résultats obtenus sont reproduits dans le tableau **I** ci-après :

**Tableau I**

| **Paramètres mesurés** | **Référence** | **Exemple 2** |
|---|---|---|
| Quantité de lanréotide dans 1 mg de gel (en mg) | 0,252 | 0,250 |
| Surface spécifique de l'acétate de lanréotide incorporé (m²/g) | 3,64 | 28,6 |
| Force nécessaire à l'injection (N) | 41,0 | 27,3 |
| Proportion dissoute après 0,5 h | 1,5 | 1,0 |
| Proportion dissoute après 1 h | 2,7 | 2,1 |
| Proportion dissoute après 2 h | 5,1 | 4,0 |
| Proportion dissoute après 3 h | 7,2 | 6,1 |
| Proportion dissoute après 4 h | 9,5 | 8,0 |
| Proportion dissoute après 24 h | 39,3 | 29,9 |
| Proportion dissoute après 48 h | 62,6 | 38,8 |
| Proportion dissoute après 72 h | 77,5 | 44,7 |
| Proportion dissoute après 96 h | 88,4 | 50,3 |

Des mesures supplémentaires ont été effectuées pour l'exemple 2 et montrent des proportions dissoutes de 57,7 % après 144 h, 66,8 % après 216 h et 77,7 % après 334 h.

On constate donc que la composition de l'exemple 2, qui se différencie de la composition de référence par sa surface spécifique presque 10 fois plus élevée, libère le peptide de façon nettement plus lente que la composition de référence. Par ailleurs, la composition de l'exemple 2 nécessite une force d'injection moindre que la composition de référence.

### Profil de libération in vivo chez le chien

La composition de référence pour ce test comporte 30 % en poids d'acétate de lanréotide de surface spécifique 0,8 m²/g (obtenu par un procédé de lyophilisation mettant en oeuvre une congélation lente), le reste de la composition consistant en de l'eau. La concentration en lanréotide pur de la composition de référence et de la composition de l'exemple 2 est donc de 246 mg par gramme de composition.
Les tests sont effectués sur deux lots de 6 chiens Beagles, chacun des chiens recevant une injection intramusculaire de 60 mg de composition de référence ou de composition de l'exemple 2.

### Résultats

Les concentrations plasmatiques mesurées pour les exemples 1 et 2 (exprimées en ng/ml) sont reportées respectivement dans le tableau **II** ci-après :

**Tableau II**

| Temps | Moyenne | | |
|---|---|---|---|
| | Réf. | | Ex. 2 |
| 0 | 0,000 | 0,000 | 0,000 |
| 0,083 h | 4,909 | 4,365 | 3,642 |
| 0,25 h | 20,930 | 11,348 | 14,591 |
| 0,5 h | 32,215 | 22,711 | 17,637 |
| 1 h | 43,215 | 26,863 | 24,847 |
| 2 h | 45,208 | 32,831 | 29,262 |
| 4 h | 44,129 | 30,112 | 29,696 |
| 8 h | 58,362 | 30,218 | 26,713 |
| 12 h | 48,041 | 20,831 | 18,235 |
| 1 jour | 29,462 | 20,771 | 16,977 |
| 2 jours | 13,677 | 7,731 | 13,105 |
| 3 jours | 9,974 | 8,000 | 12,248 |
| 4 jours | 6,683 | 6,716 | 6,520 |
| 8 jours | 3,583 | 2,564 | 3,179 |
| 11 jours | 3,225 | 2,305 | 2,513 |
| 15 jours | 1,786 | 2,280 | 2,075 |
| 18 jours | 1,305 | 2,553 | 1,481 |
| 22 jours | 1,329 | 2,317 | 1,097 |
| 25 jours | 1,182 | 1,582 | 1,605 |
| 29 jours | 1,024 | 0,983 | 0,951 |
| 32 jours | 0,685 | 0,696 | 0,924 |
| 36 jours | 0,362 | 0,486 | 0,714 |
| 39 jours | 0,194 | 0,521 | 0,792 |
| 43 jours | 0,217 | 0,443 | 0,715 |
| 46 jours | 0,189 | 0,332 | 0,768 |
| 50 jours | 0,153 | 0,337 | 0,511 |
| 53 jours | 0,148 | 0,297 | 0,513 |
| 57 jours | 0,150 | 0,228 | 0,466 |
| 60 jours | 0,131 | 0,254 | 0,411 |
| 65 jours | 0,094 | 0,191 | 0,281 |
| 72 jours | 0,093 | 0,120 | 0,312 |
| 79 jours | 0,044 | 0,147 | 0,157 |
| 86 jours | 0,063 | 0,068 | 0,200 |
| 93 jours | 0,050 | 0,072 | 0,167 |
| 100 jours | 0,046 | 0,052 | 0,137 |
| 107 jours | 0,000 | 0,064 | 0,107 |
| 114 jours | 0,000 | 0,057 | 0,062 |
| 122 jours | 0,000 | 0,034 | 0,067 |
| 128 jours | 0,000 | 0,030 | 0,048 |
| 135 jours | 0,000 | - | 0,000 |

Ces tests in vivo confirment que le pic initial (ou "burst" en anglais) est considérablement réduit pour les compositions de l'exemple 1 et de l'exemple 2 par rapport à une composition analogue comportant un peptide de surface spécifique plus faible. De plus, la libération devient trop faible après 60 jours pour la composition de référence alors qu'elle est suffisante pour assurer un taux plasmatique supérieur à 0,1 ng/ml pendant au moins 79 jours pour la composition de l'exemple 1 et au moins 107 jours pour la composition de l'exemple 2.

## Revendications

1. Composition pharmaceutique solide ou semi-solide comprenant un sel hydrosoluble et gélifiable de peptide associé éventuellement à un excipient adapté, ladite composition pharmaceutique étant **caractérisée en ce que** le sel de peptide possède une surface spécifique au moins égale à 4 m²/g et **en ce qu'**une fois injectée à un patient elle forme au contact des matières corporelles un gel chez ce patient, ledit gel étant capable de relarguer le peptide sur une durée prolongée et au moins égale à 15 jours.

2. Composition pharmaceutique selon la revendication 1, **caractérisée en ce que** le gel obtenu chez le patient est capable de relarguer le peptide sur une durée au moins égale à 1 mois.

3. Composition pharmaceutique selon la revendication 1 ou 2, **caractérisée en ce que** le gel obtenu chez le patient est capable de relarguer le peptide sur une durée au moins égale à 2 mois, et de préférence au moins égale à 3 mois.

4. Composition pharmaceutique selon l'une des revendications 1 à 3, **caractérisée en ce que** le sel de peptide possède une surface spécifique au moins égale à 5 m²/g, et de préférence au moins égale à 8 m²/g.

5. Composition pharmaceutique selon la revendication 4, **caractérisée en ce que** le sel de peptide possède une surface spécifique au moins égale à 10 m²/g, et de préférence au moins égale à 15 m²/g.

6. Composition pharmaceutique selon la revendication 5, **caractérisée en ce que** le sel de peptide possède une surface spécifique au moins égale à 20 m²/g.

7. Composition pharmaceutique selon la revendication 6, **caractérisée en ce que** le sel de peptide possède une surface spécifique au moins égale à 30 m²/g.

8. Composition pharmaceutique selon l'une des revendications 1 à 7, **caractérisée en ce qu'**un excipient est présent dans une proportion inférieure ou égale à 30 %.

9. Composition pharmaceutique selon la revendication 8, **caractérisée en ce que** l'excipient est choisi parmi un groupe de composés comprenant des polyalcools tel que le mannitol et le sorbitol, des sucres tels que le glucose et le lactose, des surfactants, des solvants organiques et des polysaccharides.

10. Composition pharmaceutique selon l'une des revendications précédentes, **caractérisée en ce qu'**elle comprend en outre de l'eau en une quantité inférieure à 50 % de la quantité nécessaire pour dissoudre entièrement le sel de peptide et adaptée pour donner une consistance semi-solide à ladite composition.

11. Composition pharmaceutique selon l'une des revendications précédentes, **caractérisée en ce que** le sel de peptide est choisi parmi les sels des substances suivantes : la triptoréline, le lanréotide, l'octréotide, un composé ayant une activité LH-RH tel que la triptoréline, la goséréline, la leuproréline, la buséréline, un antagoniste de LH-RH, un antagoniste de GPIIb/IIIa, un composé ayant une activité similaire à un antagoniste de GPIIb/IIIa, l'érythropoiétine (EPO) ou un de ses analogues, les différents interférons α, l'interféron β ou γ, la somatostatine, un dérivé de la somatostatine, un analogue de la somatostatine, l'insuline, une hormone de croissance (GH), un facteur libérateur d'hormone de croissance (GRF), un peptide libérateur d'hormone de croissance (GHRP), un facteur de croissance épidermique (EGF), une hormone mélanocyte-stimulante (MSH), une hormone libératrice de thyrotropine (TRH) ou un de ses dérivés, une hormone stimulant la thyroïde (TSH), une hormone lutéinisante (LH), une hormone stimulant les follicules (FSH), une hormone parathyroïdienne (PTH) ou un de ses dérivés, un hydrochlorure de lysozyme, un peptide relié à l'hormone parathyroïdienne (PTHrp), un fragment de peptide à N terminal (position 1→34) de l'hormone PTH humaine, la vasopressine ou un de ses dérivés, l'oxytocine, la calcitonine, un dérivé de la calcitonine ayant une activité similaire à celle de la calcitonine, un peptide relié au gène de la calcitonine (CGRP), le glucagon, un peptide similaire au glucagon (GLP), la gastrine, un peptide libérateur de gastrine (GRP), la sécrétine, la pancréozymine, la cholécystokinine, l'angiotensine, le lactogène du placenta humain, la gonadotropine chorionique humaine (HCG), l'enképhaline, un dérivé de l'enképhaline, le facteur stimulateur de colonies (CSF), l'endorphine, la kyotorphine, les interleukines, par exemple l'Interleukine 2, la tuftsine, la thymopoiétine, la thymosthymline, le facteur thymique humoral (THF), le facteur thymique sérique (FTS), un dérivé du facteur thymique sérique (FTS), la thymosine, le facteur thymique X, le facteur de nécrose tumorale (TNF), la motiline, la bombésine ou un de ses dérivés, la prolactine, la neurotensine, la dynorphine, la caeruléine, la substance P, l'urokinase, l'asparaginase, la bradykinine, la kallikréine, le facteur de croissance nerveuse, un facteur de coagulation sanguine, la polymixine B, la colistine, la gramicidine, la bacitracine, un peptide stimulant la synthèse protéique, un antagoniste de l'endothéline ou un de ses dérivés, un polypeptide intestinal vasoactif (VIP), l'hormone adrénocorticotropique (ACTH) ou un de ses fragments, un facteur de croissance dérivé des plaquettes (PDGF), une protéine morphogénétique osseuse (BMP), un polypeptide activant l'adénylatecyclase pituitaire (PACAP), le neuropeptide Y (NPY), le peptide YY (PYY), un polypeptide inhibiteur gastrique (GIP).

12. Composition pharmaceutique selon la revendication 11, **caractérisée en ce que** le peptide est choisi dans un groupe comprenant des sels de la somatostatine ou de ses analogues, en particulier l'acétate de lanréotide ou l'acétate d'octréotide, des sels de la triptoréline, en particulier l'acétate de triptoréline, des sels de la calcitonine ou de ses analogues, des sels d'analogues de l'hormone LH-RH, des sels des hormones GH, GRF, PTH ou du peptide PTHrp, et des analogues de ces derniers.

13. Composition pharmaceutique selon la revendication 11 ou 12, **caractérisée en ce que** le peptide est l'acétate de triptoréline.

14. Composition pharmaceutique selon la revendication 11 ou 12, **caractérisée en ce que** le peptide est l'acétate de lanréotide ou l'acétate d'octréotide.

## Claims

1. Solid or semi-solid pharmaceutical composition comprising a hydrosoluble and gellable peptide salt optionally combined with an appropriate excipient, said pharmaceutical composition being **characterized in that** the peptide salt has a specific surface area at least equal to 4 m²/g and **in that** once injected into a patient it forms on contact with body matter a gel in this patient, said gel being capable of releasing the peptide over a prolonged period of at least 15 days.

2. Pharmaceutical composition according to claim 1, **characterized in that** the gel obtained in the patient is capable of releasing the peptide over a period equal to at least 1 month.

3. Pharmaceutical composition according to claim 1 or 2, **characterized in that** the gel obtained in the patient is capable of releasing the peptide over a period equal to at least 2 months and preferably equal to at least 3 months.

4. Pharmaceutical composition according to one of claims 1 to 3, **characterized in that** the peptide salt has a specific surface area equal to at least 5 m²/g, and preferably equal to at least 8 m²/g.

5. Pharmaceutical composition according to claim 4, **characterized in that** the peptide salt has a specific surface area equal to at least 10 m²/g, and preferably equal to at least 15 m²/g.

6. Pharmaceutical composition according to claim 5, **characterized in that** the peptide salt has a specific surface area equal to at least 20 m²/g.

7. Pharmaceutical composition according to claim 6, **characterized in that** the peptide salt has a specific surface area equal to at least 30 m²/g.

8. Pharmaceutical composition according to one of claims 1 to 7, **characterized in that** an excipient is present in a proportion less than or equal to 30 %.

9. Pharmaceutical composition according to claim 8, **characterized in that** the excipient is chosen from a group of compounds comprising polyalcohols such as mannitol and sorbitol, sugars such as glucose and lactose, surfactants, organic solvents and polysaccharides.

10. Pharmaceutical composition according to one of the previous claims, **characterized in that** it also comprises water in a quantity which is less than 50% of the quantity needed to dissolve the peptide salt completely, and which is adapted to give said composition a semi-solid consistency.

11. Pharmaceutical composition according to one of the previous claims, **characterized in that** the peptide salt is chosen from the salts of the following substances: triptorelin, lanreotide, octreotide, a compound having an LH-RH activity such as triptorelin, goserelin, leuprorelin, buserelin, an LH-RH antagonist, a GPIIb/IIIa antagonist, a compound having an activity similar to a GPIIb/IIIa antagonist, erythropoietin (EPO) or one of its analogues, the different α interferons, β or γ interferon, somatostatin, a somatostatin derivative, an analogue of somatostatin, insulin, a growth hormone (GH), a growth hormone releasing factor (GHRF), a growth hormone releasing peptide (GHRP), an epidermal growth factor (EGF), a melanocyte stimulating hormone (MSH), a thyrotropin releasing hormone (TRH) or one of its derivatives, a thyroid stimulating hormone (TSH), a luteinizing hormone (LH), a follicle stimulating hormone (FSH), a parathyroid hormone (PTH) or one of its derivatives, a lysozyme hydrochloride, a parathyroid hormone related peptide (PTHrp), an N-terminal peptide fragment (position 1→34) of human PTH hormone, vasopressin or one of its derivatives, oxytocin, calcitonin, a calcitonin derivative with a similar activity to that of calcitonin, a calcitonin gene related peptide (CGRP), glucagon, a peptide similar to glucagon (GLP), gastrin, a gastrin releasing peptide (GRP), secretin, pancreozymin, cholecystokinin, angiotensin, human placental lactogen, human chorionic gonadotropin (HCG), enkephalin, an enkephalin derivative, colony stimulating factor (CSF), endorphin, kyotorphin, interleukins, for example interleukin-2, tuftsin, thymopoietin, thymostimulin, thymic humoral factor (THF), thymic serum factor (TSF), a derivative of thymic serum factor (TSF), thymosin, thymic factor X, tumour necrosis factor (TNF), motilin, bombesin or one of its derivatives, prolactin, neurotensin, dynorphin, caerulein, substance P, urokinase, asparaginase, bradykinin, kallikrein, nerve growth factor, a blood clotting factor, polymixin B, colistin, gramicidin, bacitracin, a protein synthesis stimulating peptide, an endothelin antagonist or one of its derivatives, a vasoactive intestinal polypeptide (VIP), adrenocorticotropic hormone (ACTH) or one of its fragments, a platelet derived growth factor (PDGF), a bone morphogenetic protein (BMP), a pituitary adenylate cyclase activating polypeptide (PACAP), neuropeptide Y (NPY), peptide YY (PVY) and a gastric inhibitory polypeptide (GIP).

12. Pharmaceutical composition according to claim 11, **characterized in that** the peptide is selected from a group comprising salts of somatostatin or its analogues, particularly lanreotide acetate or octreotide acetate, triptorelin salts, in particular triptorelin acetate, salts of calcitonin or its analogues, salts of LH-RH hormone analogues, salts of GH, GHRF, PTH hormones or PTHrp peptide, and analogues of the latter.

13. Pharmaceutical composition according to claim 11 or 12, **characterized in that** the peptide is triptorelin acetate.

14. Pharmaceutical composition according to claim 11 or 12, **characterized in that** the peptide is lanreotide acetate or octreotide acetate.

## Patentansprüche

1. Feste oder halbfeste pharmazeutische Zusammensetzung, umfassend ein wasserlösliches und gelierbares Peptidsalz, dem ggf. ein angepasster Trägerstoff zugeordnet ist, wobei diese pharmazeutische Zusammensetzung **dadurch gekennzeichnet ist, daß** das Peptidsalz eine spezifische Oberfläche von mindestens gleich 4 m²/g aufweist und daß sie nach Injizierung bei einem Patienten im Kontakt mit den Körpersubstanzen bei diesem Patienten ein Gel bildet, das das Peptid über einen längeren Zeitraum von mindestens gleich 15 Tagen aussalzen kann.

2. Pharmazeutische Zusammensetzung nach Anspruch 1, **dadurch gekennzeichnet, daß** das beim Patienten erhaltene Gel das Peptid über einen Zeitraum von mindestens gleich 1 Monat aussalzen kann.

3. Pharmazeutische Zusammensetzung nach Anspruch 1 oder 2, **dadurch gekennzeichnet, daß** das beim Patienten erhaltene Gel das Peptid über einen Zeitraum von mindestens gleich 2 Monaten und vorzugsweise von mindestens gleich 3 Monaten aussalzen kann.

4. Pharmazeutische Zusammensetzung nach einem der Ansprüche 1 bis 3, **dadurch gekennzeichnet, daß** das Peptidsalz eine spezifische Oberfläche von mindestens gleich 5 m²/g und vorzugsweise von mindestens gleich 8 m²/g aufweist.

5. Pharmazeutische Zusammensetzung nach Anspruch 4, **dadurch gekennzeichnet, daß** das Peptidsalz eine spezifische Oberfläche von mindestens gleich 10 m²/g und vorzugsweise von mindestens gleich 15 m²/g aufweist.

6. Pharmazeutische Zusammensetzung nach Anspruch 5, **dadurch gekennzeichnet, daß** das Peptidsalz eine spezifische Oberfläche von mindestens gleich 20 m²/g aufweist.

7. Pharmazeutische Zusammensetzung nach Anspruch 6, **dadurch gekennzeichnet, daß** das Peptidsalz eine spezifische Oberfläche von mindestens gleich 30 m²/g aufweist.

8. Pharmazeutische Zusammensetzung nach einem der Ansprüche 1 bis 7, **dadurch gekennzeichnet, daß** ein Trägerstoff in einem Anteil von weniger als oder gleich 30% vorhanden ist.

9. Pharmazeutische Zusammensetzung nach Anspruch 8, **dadurch gekennzeichnet, daß** der Trägerstoff aus einer Gruppe von Verbindungen ausgewählt ist, die Polyalkohole, wie z.B. Mannit und Sorbit, Zucker, wie z.B. Glucose und Lactose, Tenside, organische Lösungsmittel und Polysaccharide umfaßt.

10. Pharmazeutische Zusammensetzung nach einem der vorhergehenden Ansprüche, **dadurch gekennzeichnet, daß** sie außerdem Wasser in einer Menge von weniger als 50% der Menge umfaßt, die erforderlich ist, um das Peptidsalz vollständig aufzulösen, und die so eingestellt ist, daß der Zusammensetzung eine halbfeste Konsistenz verliehen wird.

11. Pharmazeutische Zusammensetzung nach einem der vorhergehenden Ansprüche, **dadurch gekennzeichnet, daß** das Peptidsalz aus den Salzen der folgenden Substanzen ausgewählt ist: Triptorelin, Lanreotid, Octreotid, eine Verbindung mit LHRH-Aktivität, wie z.B. Triptorelin, Goserelin, Leuprorelin, Buserelin, ein LHRH-Antagonist, ein GPIIb/IIIa-Antagonist, eine Verbindung mit einer Aktivität ähnlich einem GPIIb/IIIa-Antagonisten, Erythropoietin (EPO) oder eines seiner Analogen, die verschiedenen Interferone-alpha, Interferon-beta oder -gamma, Somatostatin, ein Derivat von Somatostatin, ein Analoges von Somatostatin, Insulin, ein Wachstumshormon (GH), ein Wachstumshormon-freisetzender Faktor (GRF), ein Wachstumshormon-freisetzendes Peptid (GHRP), ein Hautwachstumsfaktor (EGF), ein Melanozytenstimulierendes Hormon (MSH), ein Thyrotropin-freisetzendes Hormon (THR) oder eines seiner Derivate, Thyroid-stimulierendes Hormon (THS), ein luteinisierendes Hormon (LH), ein Follikel-stimulierendes Hormon (FSH), ein Parathyroid-Hormon (PTH) oder eines seiner Derivate, ein Lysozym-Hydrochlorid, ein mit dem Parathyroid-Hormon verwandtes Peptid (PTHrp), ein Peptidfragment mit endständigem N (Position 1 ---> 34) des menschlichen PTH-Hormons, Vasopressin oder eines seiner Derivate, Oxytocin, Calcitonin, ein Derivat von Calcitonin mit einer ähnlichen Aktivität wie Calcitonin, ein mit dem Calcitonin-Gen verwandtes Peptid (CGRP), Glucagon, ein Glucagon-ähnliches Peptid (GLP), Gastrin, ein Gastrin-freisetzendes Peptid (GRP), Secretin, Pancreozymin, Cholecystokinin, Angiotensin, Lactogen der menschlichen Plazenta, menschliches Chorion-Gonadotropin (HCG), Enkephalin, ein Enkephalin-Derivat, der koloniestimulierende Faktor (CSF), Endorphin, Kyotorphin, Interleukine, z.B. Interleukin 2, Tuftsin, Thymopoietin, Thymosthymlin, der Thymushumoralfaktor (THF), der Thymus-Serum-Faktor (FTS), ein Derivat des Thymus-Serum-Faktors (FTS), Thymosin, der Thymusfaktor X, der Tumornekrosefaktor (TNF), Motilin, Bombesin oder eines seiner Derivate, Prolactin, Neurotensin, Dynorphin, Caerulein, die Substanz P, Urokinase, Asparaginase, Bradykinin, Kallikrein, der Nervenwachstumsfaktor, ein Blutgerinnungsfaktor, Polymixin B, Colistin, Gramicidin, Bacitracin, ein die Proteinsynthese stimulierendes Peptid, ein Endothelin-Antagonist oder eines seiner Derivate, eine vasoaktives Intestinal-Polypeptid (VIP), das adrenokortikotropische Hormon (ACTH) oder eines seiner Fragmente, ein von Blutplättchen abgeleiteter Wachstumsfaktor (PDGF), ein knochenmorphogenetisches Protein (BMP), ein die pituitäre Adenylatecyclase aktivierendes Polypeptid (PACAP), das Neuropeptid Y (NPY), das Peptid YY (PYY), ein gastroinhibitorisches Polypeptid (GIP).

12. Pharmazeutische Zusammensetzung nach Anspruch 11, **dadurch gekennzeichnet, daß** das Peptid aus einer Gruppe ausgewählt ist, die Salze von Somatostatin oder seiner Analogen, insbesondere Lanreotidacetat oder Octreotidacetat, Salze von Triptorelin, insbesondere Triptorelinacetat, Salze von Calcitonin oder seiner Analogen, Salze von Analogen des LHRH-Hormons, Salze der Hormone GH, GRF, PTH oder des Peptids PTHrp und von deren Analogen umfaßt.

13. Pharmazeutische Zusammensetzung nach Anspruch 11 oder 12, **dadurch gekennzeichnet, daß** das Peptid Triptorelinacetat ist.

14. Pharmazeutische Zusammensetzung nach Anspruch 11 oder 12, **dadurch gekennzeichnet, daß** das Peptid Lanreotidacetat oder Octreotidacetat ist.
